(19) Europäisches Patentamt
European Patent Office
Office européen des brevets

(11) Veröffentlichungsnummer: **0 300 344 B1**

(12) **EUROPÄISCHE PATENTSCHRIFT**

(45) Veröffentlichungstag der Patentschrift: **06.10.93**

(21) Anmeldenummer: **88111192.6**

(22) Anmeldetag: **13.07.88**

(51) Int. Cl.$^5$: **C07D 285/08**, C07D 285/12, C07D 417/12, C07C 233/88, C07C 235/38, C07C 239/04, A01N 43/02

Die Akte enthält technische Angaben, die nach dem Eingang der Anmeldung eingereicht wurden und die nicht in dieser Patentschrift enthalten sind.

# (54) Halogenierte Thiadiazolyl-oxyessig-säureamide, Verfahren und Zwischenprodukte zu ihrer Herstellung und ihre Verwendung als Herbizide.

(30) Priorität: **23.07.87 DE 3724359**
**24.07.87 DE 3724467**
**04.08.87 DE 3725849**
**08.06.88 DE 3819477**

(43) Veröffentlichungstag der Anmeldung:
**25.01.89 Patentblatt 89/04**

(45) Bekanntmachung des Hinweises auf die Patenterteilung:
**06.10.93 Patentblatt 93/40**

(84) Benannte Vertragsstaaten:
**BE CH DE FR GB IT LI NL**

(56) Entgegenhaltungen:
**EP-A- 0 018 497** **EP-A- 0 029 171**
**EP-A- 0 081 730** **EP-A- 0 094 541**
**EP-A- 0 100 038** **EP-A- 0 100 044**
**EP-A- 0 192 117** **DE-A- 2 310 757**

(73) Patentinhaber: **BAYER AG**

**D-51368 Leverkusen(DE)**

(72) Erfinder: **Förster, Heinz, Dr.**
**Am Eckbusch 47**
**D-5600 Wuppertal 1(DE)**
Erfinder: **Andree, Roland, Dr.**
**Schillerstrasse 17**
**D-4018 Langenfeld(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Strang, Harry, Dr.**
**Unterdorfstrasse 6 a**
**D-4000 Düsseldorf 31(DE)**

**EP 0 300 344 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue halogenierte Thiadiazolyloxyessigsäureamide, ein Verfahren und neue Zwischenprodukte zu ihrer Herstellung und ihre Verwendung als Herbizide.

Es ist bereits bekannt, daß bestimmte Heteroaryloxyessigsäureamide, wie z. B. 2-(Benzthiazol-2-yl-oxy)-essigsäure-N-methyl-anilid und 2-(4,5-Dichlor-thiazol-2-yl-oxy)-essigsäure-N-methyl-anilid,herbizide Eigenschaften aufweisen (vgl. US-P 4 509 971 und US-P 4 645 525 / EP-A-0 018 497).

Aus der Stoffklasse der herbizid wirksamen Heteroaryloxyacetamide sind weitere strukturverwandte Verbindungen vorbekannt, so z.B. bestimmte Azolyloxy-carbonsäure-N-oxy-amide (vgl. EP-A-0 029 171), substituierte 5-Trifluormethyl-1,3,4-thiadiazol-2-yl-oxyessigsäureamide und substituierte 3-Trihalogenmethyl-1,2,4-thiadiazol-5-yl-oxyessigsäureamide (vgl. EP-A-0 094 541, EP-A-0 100 038 und EP-A-0 100 044) sowie - als nächster Stand der Technik - 5-Chlor-1,3,4-thiadiazol-2-yloxyacetamide (vgl. EP-A-0 192 117).

Die herbizide Wirksamkeit dieser vorbekannten Verbindungen gegenüber Schadpflanzen bzw. ihre Selektivität in Nutzpflanzen ist jedoch nicht immer ganz zufriedenstellend.

Es wurden nun neue halogenierte Thiadiazolyl-oxyessigsäureamide der allgemeinen Formel (I) gefunden,

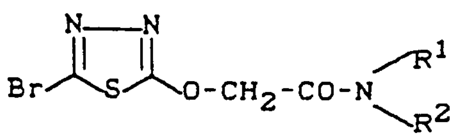

(I)

in welcher

$R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,

$R^2$ für $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$- oder $C_6$-Cycloalkenyl, für Benzyl, welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder

$R^1$ und $R^2$ zusammen mit dem Stickstoffatom, an das sie gebunden sind, einen gegebenenfalls einbis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist.

Weiter wurde gefunden, daß man die neuen halogenierten Thiadiazolyl-oxyessigsäureamide der allgemeinen Formel (I) erhält, wenn man Thiadiazolderivate der allgemeinen Formel (II)

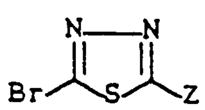

(II)

in welcher

Z für eine nucleofuge Abgangsgruppe steht,

mit Hydroxyessigsäureamiden der allgemeinen Formel (III)

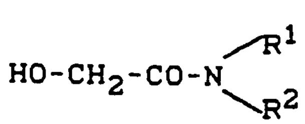

(III)

in welcher

$R^1$ und $R^2$ die oben angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen halogenierten Thiadiazolyl-oxyessigsäureamide der allgemeinen Formel (I) interessante herbizide Eigenschaften besitzen.

Überraschenderweise zeigen die neuen halogenierten Thiadiazolyl-oxyessigsäureamide der allgemeinen Formel (I) erheblich stärkere herbizide Wirkung gegen verbreitete, schwer bekämpfbare Unkräuter als die oben genannten Verbindungen bei guter Verträglichkeit gegenüber wichtigen Kulturpflanzen.

Gegenstand der Erfindung sind vorzugsweise Verbindungen der Formel (I), in welcher

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy, Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydro-chinolinyl stehen.

Beipiele für die Verbindungen der Formel (I) sind in der nachstehenden Tabelle 1 aufgeführt.

**<u>Tabelle 1:</u> Beispiele für die Verbindungen der Formel (I)**

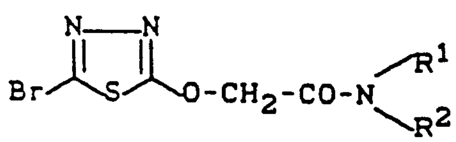

(I)

**Tabelle 1**

| $R^1$ | $R^2$ | bzw. 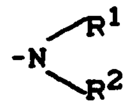 |
|---|---|---|
| $-C_4H_9-n$ | $-C_4H_9-n$ | |
| $-C_3H_7-n$ | $-C_3H_7-n$ | |
| $-CHCH_2CH_3$<br>$CH_3$ | $-OCH_3$ | |
| $-CH(CH_3)_2$ | $-OCH(CH_3)_2$ | |
| $-CH(CH_3)_2$ | $-OCH_2CH_2OC_2H_5$ | |
| $-CH_3$ | 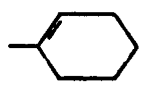 | |
| $-C_2H_5$ | $-C_2H_5$ | |
| $-CH_3$ | 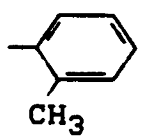 | |
| $-CH_3$ | 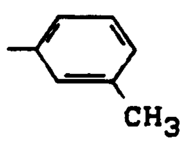 | |
| $-CH_3$ | 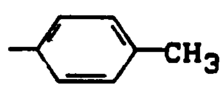 | |
| $-CH(CH_3)_2$ | 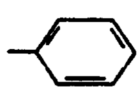 | |
| $-CH(CH_3)_2$ | 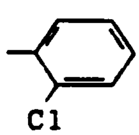 | |

**Tabelle 1** - Fortsetzung

| $R^1$ | $R^2$ | bzw. 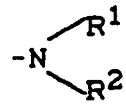 |
|---|---|---|
| $-CH(CH_3)_2$ | 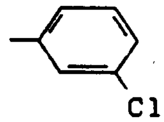 | |
| $-CH(CH_3)_2$ | 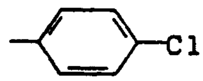 | |
| $-CH(CH_3)_2$ | 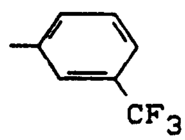 | |
| $-CH_3$ | 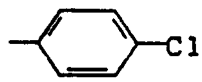 | |
| $-CH_3$ | 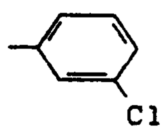 | |
| $-CH_3$ | 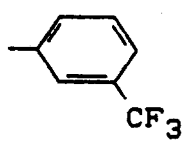 | |

Verwendet man beispielsweise 2,5-Dibrom-1,3,4-thiadiazol und Hydroxyessigsäurepiperidid als Ausgangsstoffe, so läßt sich der Reaktionsablauf beim erfindungsgemäßen Verfahren durch das folgende Formelschema darstellen:

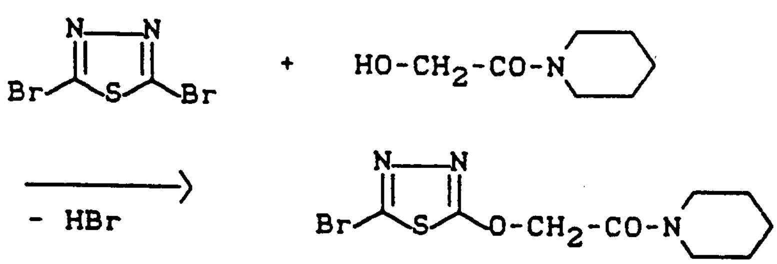


Die zur Durchführung des erfindungsgemäßen Verfahrens als Ausgangsstoffe zu verwendenden Thiadiazolderivate sind durch die Formel (II) allgemein definiert. In Formel (II) steht Z vorzugsweise für Halogen oder $C_1$-$C_4$-Alkylsulfonyl, insbesondere für Chlor, Brom oder Methylsulfonyl.

Die Thiadiazolderivate der Formel (II) sind bekannt und/oder können nach an sich bekannten Verfahren hergestellt werden (vgl. J. Org. Chem. 38 (1973), 465 - 471; DE-OS 24 32 005).

Die weiter als Ausgangsstoffe zu verwendenden Hydroxyessigsäureamide sind durch die Formel (III) allgemein definiert, In Formel (III) haben $R^1$ und $R^2$ vorzugsweise diejenigen Bedeutungen, die bereits oben im Rahmen der Beschreibung der erfindungsgemäßen Verbindungen der Formel (I) als bevorzugt für $R^1$ bzw. $R^2$ angegeben wurden.

Beispiele für die Ausgangsstoffe der Formel (III) sind in der nachstehenden Tabelle 2 aufgeführt.

**<u>Tabelle 2</u>: Beispiele für die Ausgangsstoffe der Formel (III)**

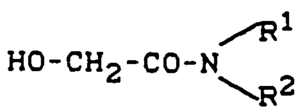

(III)

| $R^1$ | $R^2$ | $R^1$ | $R^2$ |
|---|---|---|---|
| $CH_3$ | $OCH(CH_3)_2$ | $CH_3$ | $OCH_2CH_2OC_2H_5$ |
| $CH_3$ | $CH(CH_3)_2$ | $CH_3$ | $CH_2CH(CH_3)_2$ |
| $CH_3$ | 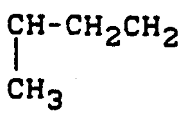 | $C_2H_5$ | $OC_2H_5$ |
| $C_2H_5$ | $OCH_2CH_2OC_2H_5$ | $C_2H_5$ | $OCH_2CH(CH_3)_2$ |

Tabelle 2 - Fortsetzung

| $R^1$ | $R^2$ | $R^1$ | $R^2$ |
|---|---|---|---|
| $CH_3$ | 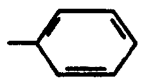 | $CH_3$ | 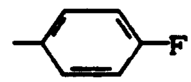 |
| $CH_3$ | 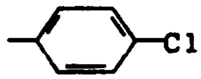 | $CH_3$ | 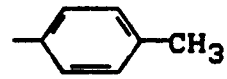 |
| $CH_3$ | 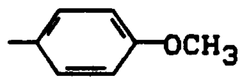 | $CH_3$ | 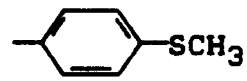 |
| $CH_3$ | 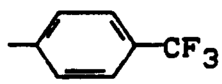 | $CH_3$ | 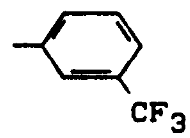 |
| $CH_3$ | 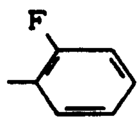 | $CH_3$ | 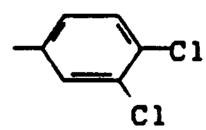 |
| $CH_3$ | 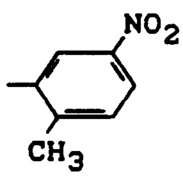 | $C_2H_5$ | 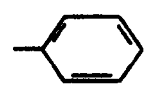 |
| $CH_3$ | 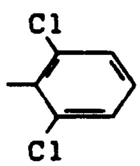 | $CH_3$ | 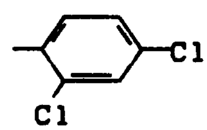 |
| $CH_3$ | 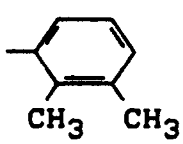 | $CH_3$ | 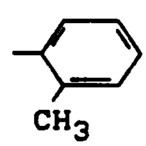 |
| $CH_3$ | 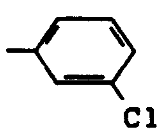 | $CH_3$ | 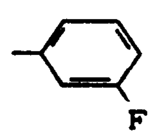 |

<u>Tabelle 2</u> - Fortsetzung

| $R^1$ | $R^2$ | $R^1$ | $R^2$ |
|---|---|---|---|
| $CH_3$ | 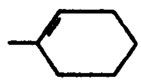 | $CH_3$ | $CH_3$ |
| $CH(CH_3)_2$ | 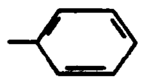 | $C_2H_5$ | 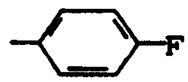 |
| $C_2H_5$ | 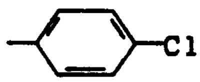 | $C_2H_5$ | 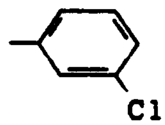 |
| $CH(CH_3)_2$ | 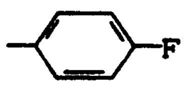 | $CH(CH_3)_2$ | 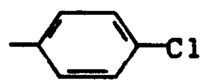 |
| $CH(CH_3)_2$ | 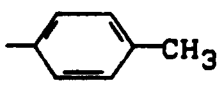 | $CH(CH_3)_2$ | 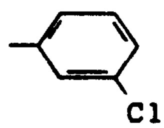 |
| $CH(CH_3)_2$ | 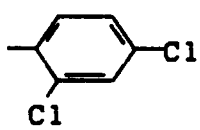 | $CH(CH_3)_2$ | 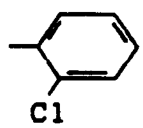 |
| $C_2H_5$ | $C_2H_5$ | $C_3H_7$ | $C_3H_7$ |
| $CH(CH_3)_2$ | $CH(CH_3)_2$ | $CH(CH_3)_2$ | $OCH_3$ |
| $CH(CH_3)_2$ | $OCH_2CH_2OC_2H_5$ | $CH(CH_3)_2$ | $OC_2H_5$ |
| $CH(CH_3)_2$ | $OC_3H_7$ | $CH_3$ | $OCH_2CH(CH_3)_2$ |
| $CH_3$ | $CH_2OCH_3$ | $C_4H_9$ | $C_4H_9$ |
| $C_2H_5$ | $OCH_3$ | $CH_3$ | $CH_2CF_3$ |
| $CH_2CH{=}CH_2$ | $CH_2CH{=}CH_2$ | $CH_2C{\equiv}CH$ | $CH_2C{\equiv}CH$ |
| $CH_3$ | $CHC{\equiv}CH$ | $CH(CH_3)CH_2CH_3$ | $OCH_3$ |

**Tabelle 2** - Fortsetzung

| $R^1$ | $R^2$ | $R^1$ | $R^2$ |
|---|---|---|---|
| 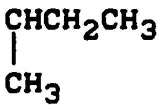 | $OCH_3$ | $CH_3$ | 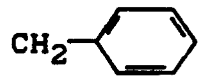 |
| $C_2H_5$ | 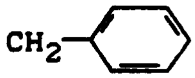 | $CH(CH_3)_2$ | 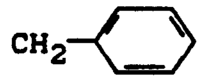 |
| $C_2H_5$ | $CH_2C{\equiv}CH$ | $CH(CH_3)_2$ | $CH_2{\equiv}CH$ |
| $CH(CH_3)_2$ | $OCH(CH_3)_2$ | | |

| 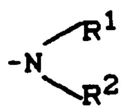 | 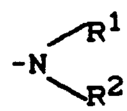 |
|---|---|
| 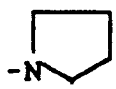 | 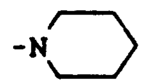 |
| 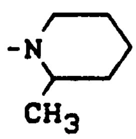 | 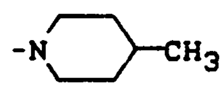 |
| 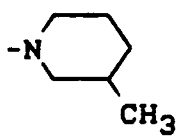 | 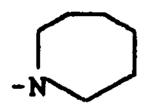 |
| 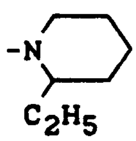 | 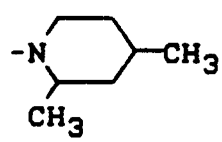 |
| 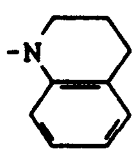 | |

Die Hydroxyessigsäureamide der Formel (III) sind bekannt und/oder können nach an sich bekannten Verfahren hergestellt werden (vgl. US-P 4 509 971 und US-P 4 645 525; ferner US-P 4 334 073, DE-A 30 38 598, DE-A 30 38 636).

Teilweise neu und von besonderem Interesse sind die Hydroxyessigsäureamide der allgemeinen Formel (IIIa)

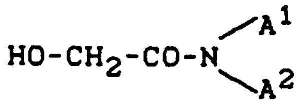

(IIIa)

in welcher

$A^1$ für Isopropyl steht und

$A^2$ für einfach oder zweifach, gleichartig oder verschiedenartig durch Fluor, Chlor, Brom, $C_1$-$C_4$-Alkyl, Trifluormethyl, $C_1$-$C_4$-Alkoxy und/oder $C_1$-$C_4$-Alkylthio substituiertes Phenyl steht.

Beispiele für die Hydroxyessigsäureamide der Formel (IIIa) sind nachstehend aufgeführt:

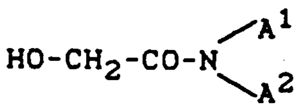

(IIIa)

$A^1$: $-CH(CH_3)_2$

$A^2$:

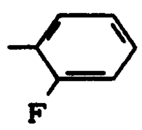, 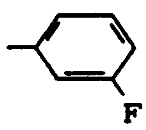, 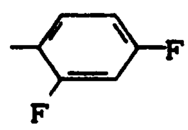, 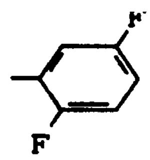, 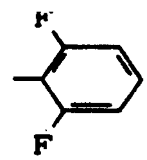,

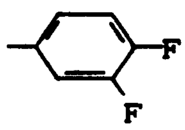, 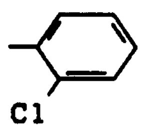, 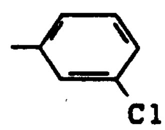, 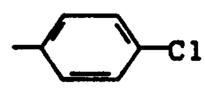,

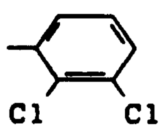, 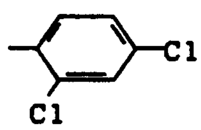, 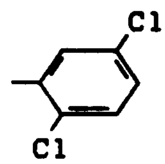, 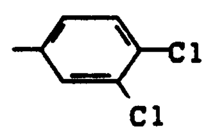,

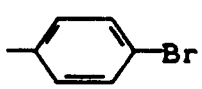, 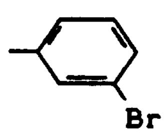, 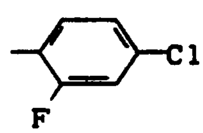, 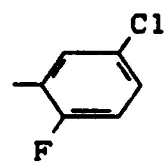,

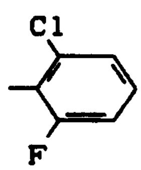, 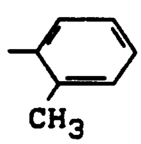, 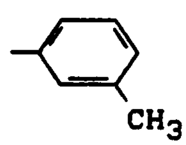, 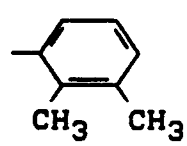,

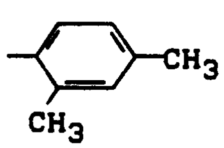, 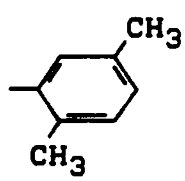, 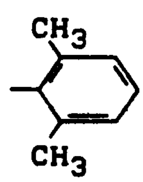, 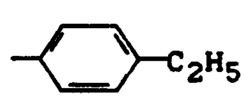,

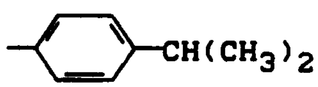, 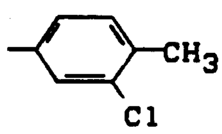, 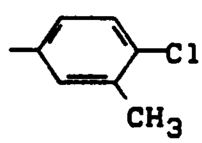,

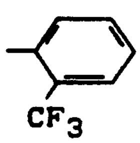, 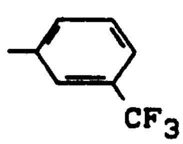, 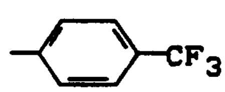, 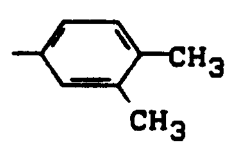,

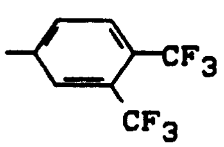, 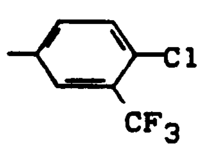, 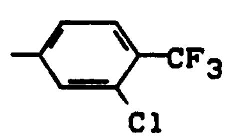, 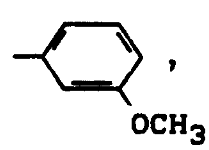,

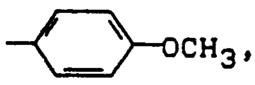, 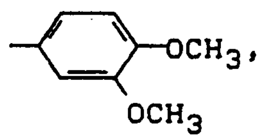, 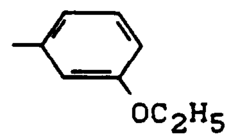,

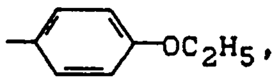, 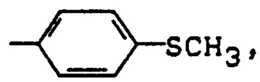, 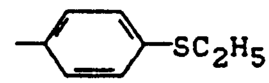.

Man erhält die Hydroxyessigsäureamide der Formel (IIIa), wenn man entsprechende Acetoxyessigsäureamide der allgemeinen Formel (IV)

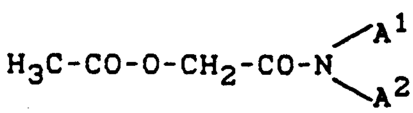 (IV)

in welcher

$A^1$ und $A^2$ die oben angegebenen Bedeutungen haben,

mit Alkalimetall-hydroxiden, wie z. B. Lithium-, Natrium- oder Kalium-hydroxid, und/oder mit Alkalimetallalkoholaten, wie z. B. Natrium-methylat, Natrium-ethylat, Kalium-methylat oder Kalium-ethylat, in Gegenwart von Wasser und/oder Alkoholen, wie z. B. Methanol oder Ethanol, bei Temperaturen zwischen 0 °C und 80 °C umsetzt und nach üblichen Methoden aufarbeitet.

Die als Zwischenprodukte benötigten Acetoxyessigsäureamide der allgemeinen Formel (IV) sind noch nicht aus der Literatur bekannt.

Man erhält die neuen Actoxyessigsäureamide der Formel (IV), wenn man Amine der allgemeinen Formel (V)

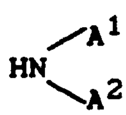 (V)

in welcher

$A^1$ und $A^2$ die oben angegebenen Bedeutungen haben,

mit Acetoxyacetylchlorid (vgl. US-P 4 509 971) in Gegenwart eines Säureakzeptors, wie z. B. Pyridin, und gegebenenfalls in Gegenwart eines Verdünnungsmittels, wie z. B. Toluol, bei Temperaturen zwischen -20 °C und +50 °C umsetzt und nach üblichen Methoden aufarbeitet.

Die als Zwischenprodukte benötigten Amine der allgemeinen Formel (V) sind bekannt und/oder können nach an sich bekannten Verfahren hergestellt werden (vgl. Herstellungsbeispiele).

Das erfindungsgemäße Verfahren zur Herstellung der neuen halogenierten Thiadiazolyloxyessigsäureamide der Formel (I) wird vorzugsweise unter Verwendung von Verdünnungsmitteln durchgeführt. Hierzu gehören vorzugsweise Kohlenwasserstoffe, wie z. B. Toluol, Xylol oder Cyclohexan, Halogenkohlenwasserstoffe, wie z. B. Methylenchlorid, Ethylenchlorid, Chloroform oder Chlorbenzol, Ether, wie z. B. Diethylether, Dipropylether, Diisopropylether, Dibutylether, Diisobutylether, Glycoldimethylether, Tetrahydrofuran und Dioxan, Alkohole, wie z. B. Methanol, Ethanol, Propanol, Isopropanol oder Butanol, Ketone, wie z. B. Aceton, Methylethylketon, Methylisopropylketon und Methylisobutylketon, Ester, wie z. B. Essigsäuremethylester und Essigsäureethylester, Amide, wie z. B. Dimethylformamide. Dimethylacetamid und N-Methyl-pyrrolidon, Nitrile, wie z. B. Acetonitril und Propionitril Sulfoxide, wie z. B. Dimethylsulfoxid sowie Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhaft unter Verwendung von Säurebindemitteln durchgeführt. Als solche werden vorzugsweise stark basische Alkali- und Erdalkalimetallverbindungen, beispielsweise Oxide, wie z. B. Natrium-, Kalium-, Magnesium- und Calciumoxid, Hydroxide, wie z. B. Natrium-, Kalium-,

Magnesium- und Calciumhydroxid und/oder Carbonate, wie z. B. Natrium-, Kalium-, Magnesium- und Calciumcarbonat verwendet.

Der Zusatz von 0,01 bis 10 Gew.-% (bezogen auf eingesetztes Glycolsäureamid der Formel (III)) eines Phasentransferkatalysators mag sich in einigen Fällen als vorteilhaft erweisen. Als Beispiele für solche Katalysatoren seien genannt:
Tetrabutylammoniumchlorid, Tetrabutylammoniumbromid, Tributyl-methylphosphoniumbromid, Trimethyl-$C_{13}/C_{15}$-alkyl-ammoniumchlorid, Dibenzyl-dimethyl-ammonium-methylsulfat, Dimethyl-$C_{12}/C_{14}$-alkyl-benzyl-ammoniumchlorid, Tetrabutylammoniumhydroxid, 18-Krone-6, Triethylbenzylammoiumchlorid, Trimethylbenzylammoniumchlorid, Tetraethylammoniumbromid.

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +100 °C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man je Mol Thiadiazolderivat der Formel (II) im allgemeinen 0,5 bis 5 Mol, vorzugsweise 0,8 bis 1,5 Mol, Hydroxyessigsäureamid der Formel (III) ein. Die Reaktionskomponenten können in beliebiger Reihenfolge zusammengegeben werden. Man rührt jeweils das Reaktionsgemisch bis zum Ende der Umsetzung und arbeitet nach üblichen Methoden auf.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopercurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe neben einer hervorrangenden Wirkung gegen Schadpflanzen gute Verträglichkeit gegenüber wichtigen Kulturpflanzen, wie z. B. Weizen, Baumwolle, Sojabohnen und Zuckerrüben, und können daher als selektive Unkrautbekämpfungsmittel eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol

oder Glykol sowie deren Ether und Ester, Ketone wie Aceton, Methylethylketone, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Supensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

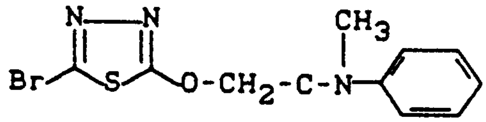

Eine Lösung von 35 g (0,14 Mol) 2,5-Dibrom-1,3,4-thiadiazol in 50 ml Acetonitril wird bei -10 °C unter Rühren zu einer Mischung aus 30,5 g (0,18 Mol) Hydroxyessigsäure-N-methyl-anilid, 11,6 g (0,18 Mol) Kaliumhydroxid-Pulver und 150 ml Isopropanol tropfenweise gegeben. Das Reaktionsgemisch wird dann 10 Stunden bei -10 °C gerührt und anschließend in ca. 200 ml Wasser gegossen. Das dabei als Öl anfallende Produkt wird durch Anreiben mit einem Glasstab zur Kristallisation gebracht, durch Absaugen isoliert und durch Umkristallisation aus Methylcyclohexan/Essigester gereinigt.

Man erhält 23 g (50 % der Theorie) 2-(5-Brom-1,3,4-thiadiazol-2-yl-oxy)-essigsäure-N-methyl-anilid vom Schmelzpunkt 101 °C.

Analog Beispiel 1 und entsprechend der allgemeinen Beschreibung des erfindungsgemäßen Herstellungsverfahrens können auch die in der nachstehenden Tabelle 3 aufgeführten Verbindungen der Formel (I) hergestellt werden.

**Tabelle 3: Beispiele für die Verbindungen der Formel (I)**

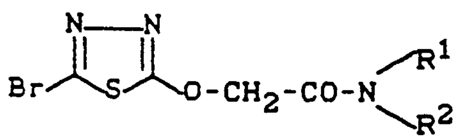

(I)

| Beisp.-Nr. | $R^1$ | $R^2$ | Schmelzpunkt (°C) bzw. Brechungsindex |
|---|---|---|---|

<u>Tabelle 3</u> - Fortsetzung

| Bsp.-<br>Nr. | $R^1$ | $R^2$ | Schmelzpunkt(°C)<br>bzw. Brechungs-<br>index |
|---|---|---|---|
| 2 | $C_4H_9$ | $C_4H_9$ | |
| 3 | $C_3H_7$ | $C_3H_7$ | |
| 4 | $CHCH_2CH_3$<br>\|<br>$CH_3$ | $OCH_3$ | |
| 5 | $CH_3$ | $CH_3$ | |
| 6 | $CH(CH_3)_2$ | $OCH_2CH_2OC_2H_5$ | |
| 7 | $CH_3$ | 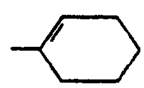 | 91 |
| 8 | $CH_3$ | $CHCH_2CH_3$<br>\|<br>$CH_3$ | |
| 9 | $CH_3$ | 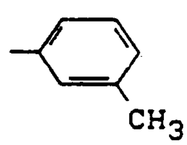 | |
| 10 | $CH_3$ | $CH_2C{\equiv}CH$ | |

**Tabelle 3** - Fortsetzung

| Bsp.-Nr. | $R^1$ | $R^2$ | Schmelzpunkt(°C) bzw. Brechungsindex |
|---|---|---|---|
| 11 | $CH_3$ | 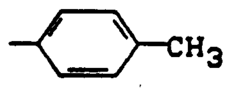 | |
| 12 | $C_2H_5$ | $C_2H_5$ | |

**Tabelle 3** - Fortsetzung

| Bsp.-Nr. | $R^1$ $R^2$ bzw. $-N(R^1)-R^2$ | Schmelzpunkt(°C) bzw. Brechungsindex |
|---|---|---|
| 13 | 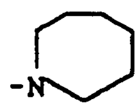 | |
| 14 | 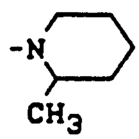 | |
| 15 | 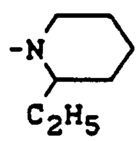 | |
| 16 | 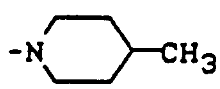 | |
| 17 | 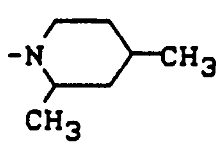 | |
| 18 | 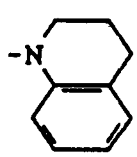 | 105 |

Tabelle 3 - Fortsetzung

| Bsp.-Nr. | $R^1$ | $R^2$ (bzw. $-N(R^1)R^2$) | Schmelzpunkt (°C) bzw. Brechungsindex |
|---|---|---|---|
| 19 | $C_2H_5$ | 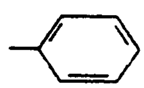 | 90 |
| 20 | $-CH(CH_3)_2$ | 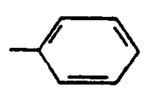 | 75 |
| 21 | $-CH(CH_3)_2$ | 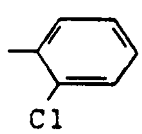 | 66 |

Tabelle 3 - Fortsetzung

| Bsp.-Nr. | $R^1$ | $R^2$ | Schmelzpunkt(°C) bzw. Brechungsindex |
|---|---|---|---|
| | bzw. $-N{<}^{R^1}_{R^2}$ | | |
| 22 | $-CH(CH_3)_2$ | 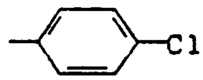 | $n_D^{20}$ = 1,5740 |
| 23 | $-CH_3$ | 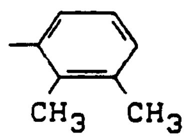 | 76 |
| 24 | $-CH_3$ | 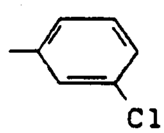 | 86 |
| 25 | $-CH(CH_3)_2$ | 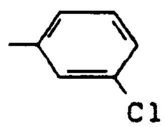 | 72 |

Ausgangsstoffe der Formel (III)

Beispiel (III-1)

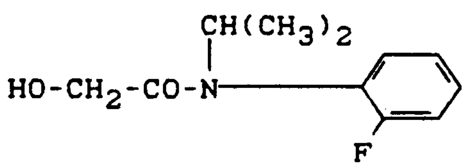

Eine Mischung aus 253 g (1.0 Mol) Acetoxyessigsäure-N-isopropyl-(2-fluor-phenyl)-amid, 44 g (1,1 Mol) Natriumhydroxid, 282 ml Wasser und 646 ml Methanol wird 3 Stunden bei 40 °C und dann 20 Stunden bei 20 °C gerührt. Zur Aufarbeitung wird durch Zugabe von 2N-Salzsäure der pH-Wert auf 6 eingestellt, nach Einengen auf etwa das halbe Volumen wird mit Methylenchlorid extrahiert, die organische Phase mit Wasser, dann mit 2N-Salzsäure und dann wieder mit Wasser gewaschen, mit Natriumsulfat getrocknet und filtriert. Vom Filtrat wird das Lösungsmittel im Wasserstrahlvakuum sorgfältig abdestilliert und der Rückstand aus Ligroin umkristallisiert.

Man erhält 123 g (58 % der Theorie) Hydroxyessigsäure-N-isopropyl-(2-fluor-phenyl)-amid vom Schmelzpunkt 51 °C.

Analog Beispiel (III-1) können auch die in der nachstehenden Tabelle 4 aufgeführten neuen Ausgangsstoffe der Formel (IIIa) hergestellt werden.

Tabelle 4: Beispiele für die neuen Ausgangsstoffe der Formel (IIIa)

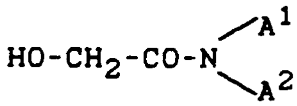 (IIIa)

| Beisp.-Nr. | $A^1$ | $A^2$ | (Kp. = Siedepunkt) Schmelzpunkt (°C) |
|---|---|---|---|
| III-2 | $-CH(CH_3)_2$ | 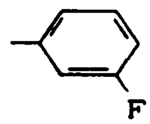 | 57 |
| III-3 | $-CH(CH_3)_2$ | 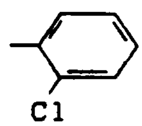 | 50 |
| III-4 | $-CH(CH_3)_2$ | 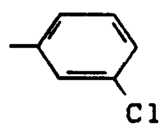 | (Kp.:140 °C/1,3Pa) |
| III-5 | $-CH(CH_3)_2$ | 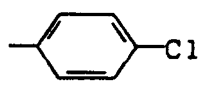 | 75 |
| III-6 | $-CH(CH_3)_2$ | 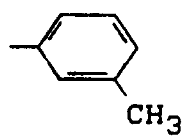 | 57 |
| III-7 | $-CH(CH_3)_2$ | 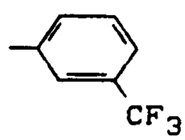 | 71 |
| III-8 | $-CH(CH_3)_2$ | 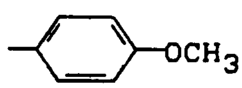 | 112 |
| III-9 | $-CH(CH_3)_2$ | 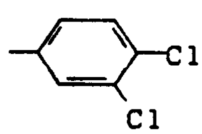 | (Kp.: 150° C/2 Pa) |
| III-10 | $-CH(CH_3)_2$ | 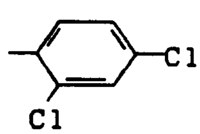 | 79 |

Ausgangsstoffe der Formel (IV)

Beispiel (IV-1)

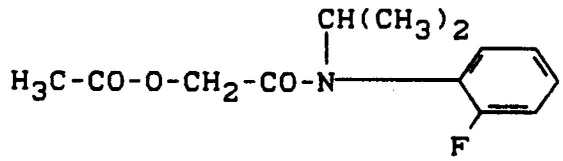

226 g (1,66 Mol) Acetoxyacetylchlorid werden langsam zu einer Mischung aus 196 g (1,28 Mol) N-Isopropyl-(2-fluor-phenyl)-amin, 157 g (1,90 Mol) Pyridin und 2 l Toluol, welche durch Kühlen mit Eis/Alkohol bie 0 °C bis 5 °C gehalten wird, gegeben. Die Reaktionsmischung wird noch eine Stunde bei 0 °C bis 5 °C und weitere 20 Stunden bei 20 °C gerührt. Zur Aufarbeitung wird auf 3 l Wasser gegossen, die organische Phase abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 256 g (79 % der Theorie) Acetoxyessigsäure-N-isopropyl-(2-fluor-phenyl)-amid als kristallinen Rückstand vom Schmelzpunkt 83 °C.

Analog Beispiel (IV-1) können auch die in der nachstehenden Tabelle 5 aufgeführten neuen Zwischenprodukte der Formel (IV) hergestellt werden.

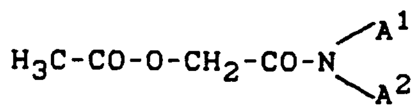

(IV)

Tabelle 5: Beispiele für die Verbindungen der Formel (IV)

| Beisp.-Nr. | $A^1$ | $A^2$ | (Brechungsindex) Schmelzpunkt (°C) |
|---|---|---|---|
| IV-2 | $-CH(CH_3)_2$ | 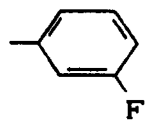 | ($n_D^{20}$: 1,5038) |
| IV-3 | $-CH(CH_3)_2$ | 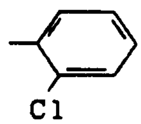 | ($n_D^{20}$: 1,5132) |
| IV-4 | $-CH(CH_3)_2$ | 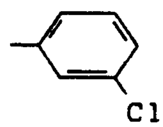 | 60 |
| IV-5 | $-CH(CH_3)_2$ | 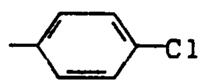 | 56 |
| IV-6 | $-CH(CH_3)_2$ | 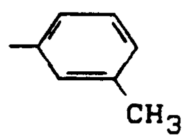 | 64 |
| IV-7 | $-CH(CH_3)_2$ | 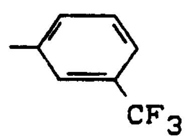 | |
| IV-8 | $-CH(CH_3)_2$ | 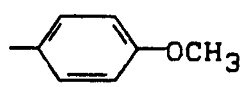 | |
| IV-9 | $-CH(CH_3)_2$ | 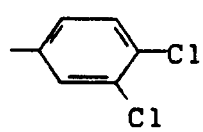 | |
| IV-10 | $-CH(CH_3)_2$ | 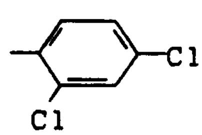 | |

Ausgangsstoffe der Formel (V)

Beispiel (V-1)

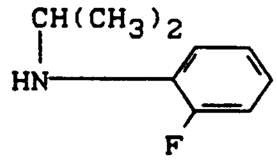

Eine Mischung aus 222 g (2 Mol) 2-Fluor-anilin, 140 g Aceton und 800 ml Eisessig wird eine Stunde bei 60 °C bis 70 °C gerührt. Dann werden bei 20 °C 94 g (2,48 Mol) Natriumboranat (Natriumtetrahydridoborat) unter Rühren innerhalb von 2 Stunden portionsweise dazugegeben. Das Reaktionsgemisch wird 90 Minuten bei 20 °C gerührt; dann werden nacheinander 3 l Eiswasser und 800 ml konzentrierte Natronlauge dazugegeben. Nach einstündigem Rühren wird mit Chloroform extrahiert, die organische Phase mit Wasser gewaschen, mit Natriumsulfat getrocknet und filtriert. Das Filtrat wird durch Vakuumdestillation aufgearbeitet.

Man erhält 198 g (65 % der Theorie) N-Isopropyl-(2-fluor-phenyl)-amin vom Siedepunkt 52 °C/40 Pa.

Analog Beispiel (V-1) können auch die in der nachstehenden Tabelle 6 aufgeführten Verbindungen der Formel (V) hergestellt werden.

**Tabelle 6:** Beispiele für die Verbindungen der Formel (V)

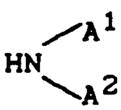

(V)

| Beisp.-Nr. | $A^1$ | $A^2$ | (Brechungsindex) Schmelzpunkt (°C) |
|---|---|---|---|
| V-2 | $-CH(CH_3)_2$ | 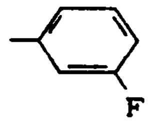 | 78 |
| V-3 | $-CH(CH_3)_2$ | 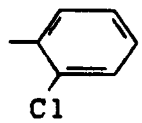 | ($n_D^{20}$: 1,5372) |
| V-4 | $-CH(CH_3)_2$ | 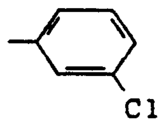 | 84 |
| V-5 | $-CH(CH_3)_2$ | 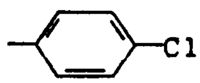 | 102 |
| V-6 | $-CH(CH_3)_2$ | 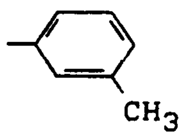 | 62 |
| V-7 | $-CH(CH_3)_2$ | 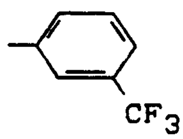 | |
| V-8 | $-CH(CH_3)_2$ | 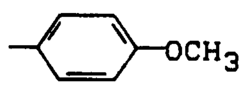 | |
| V-9 | $-CH(CH_3)_2$ | 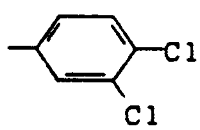 | |
| V-10 | $-CH(CH_3)_2$ | 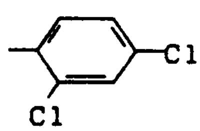 | |

Anwendungsbeispiele

Beispiel A

Pre-emergence-Test

Lösungsmittel: 5 Gewichtsteile Aceton
Emulgator; 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant, Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle, Es bedeuten:

0 % = keine Wirkung (wie unbehandelte Kontrolle)
100 % = totale Vernichtung

In diesem Test zeigen z. B. die erfindungsgemäßen Verbindungen der Formel (I) gemäß den Herstellungsbeispielen (1) und (19) bei guter Verträglichkeit in Kulturpflanzen, wie z. B. Weizen und Baumwolle, erheblich stärkere Wirkung gegen Unkräuter, wie z. B. Alopecurus, Cynodon, Digitaria, Poa und Setaria, als die bekannte Verbindung 2-(Benzthiazol-2-yl-oxy-)essigsäure-N-methyl-anilid.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen die erfindungsgemäßen Verbindungen der Formel (I) sehr gute Wirkung.

## Patentansprüche

**1.** Halogenierte Thiadiazolyl-oxyessigsäureamide der allgemeinen Formel (I)

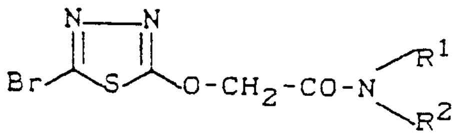

(I)

in welcher

$R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,

$R^2$ für $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$- oder $C_6$-Cycloalkenyl, für Benzyl, welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder

$R^1$ und $R^2$ zusammen mit dem Stickstoffatom, an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist.

2. Halogenierte Thiadiazolyl-oxyessigsäureamide der allgemeinen Formel (I) gemäß Anspruch 1, dadurch gekennzeichnet, daß darin
   $R^1$ für $C_1$-$C_4$-Alkyl, Allyl oder Propargyl steht,
   $R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy, Ethoxy substituiert ist), $C_1$-$C_6$-Alkoxy oder $C_1$-$C_2$-Alkoxy-$C_1$-$C_2$-alkoxy steht, oder
   $R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein-oder zweifach durch Methyl und/oder Ethyl substituiertes Pyrrolidinyl, für Perhydroazepinyl oder für 1,2,3,4-Tetrahydro-chinolinyl stehen.

3. Verfahren zur Herstellung von halogenierten Thiadiazolyl-oxyesigsäureamiden der allgemeinen Formel (I) gemäß Anspruch 1, dadurch gekennzeichnet, daß man Thiadiazolderivate der allgemeinen Formel (II)

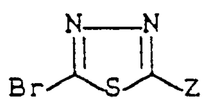

(II)

in welcher
   Z für eine nucleofuge Abgangsgruppe steht,
mit Hydroxyessigsäureamiden der allgemeinen Formel (III)

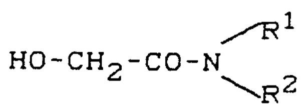

(III)

in welcher
   $R^1$ und $R^2$ die in Anspruch 1 angegebenen Bedeutungen haben,
gegebenenfalls in Gegenwart eines Verdünnungsmittels, gegebenenfalls in Gegenwart eines Säurebindemittels und gegebenenfalls in Gegenwart eines Katalysators umsetzt.

4. Herbizide Mittel, gekennzeichnet durch einen Gehalt an halogenierten Thiadiazolyl-oxyessigsäureamiden der Formel (I) gemäß Anspruch 1.

5. Verfahren zur Bekämpfung von unerwünschtem Pflanzenwachstum, dadurch gekennzeichnet, daß man halogenierte Thiadiazolyl-oxyessigsäureamide der Formel (I) gemäß Anspruch 1 auf die unerwünschten Pflanzen oder ihren Lebensraum einwirken läßt.

6. Verwendung von halogenierten Thiadiazolyl-oxyessigsäureamiden der Formel (I) gemäß Anspruch 1 zur Bekämpfung von Pflanzenwachstum.

7. Verfahren zur Herstellung von herbiziden Mitteln, dadurch gekennzeichnet, daß man halogenierte Thiadiazolyl-oxyessigsäureamide der Formel (I) gemäß Anspruch 1 mit Streckmitteln und/oder oberflächenaktiven Mitteln vermischt.

## Claims

1. Halogenated thiadiazolyl-oxyacetamides of the general formula (I)

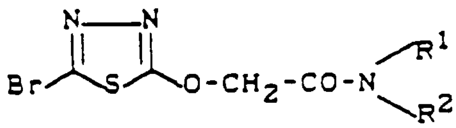

(I)

in which
$R^1$ represents hydrogen or $C_1$-$C_8$-alkyl, which is optionally substituted by fluorine, chlorine, cyano or $C_1$-$C_4$-alkoxy, or represents $C_2$-$C_8$-alkenyl which is optionally substituted by fluorine and/or chlorine, or represents $C_2$-$C_8$-alkinyl, or represents benzyl,
$R^2$ represents $C_1$-$C_8$-alkyl which is optionally substituted by fluorine, chlorine, cyano or $C_1$-$C_4$-alkoxy, or represents $C_2$-$C_8$-alkenyl which is optionally substituted by fluorine and/or chlorine, or represents $C_2$-$C_8$-alkinyl, or represents $C_3$-$C_6$-cycloalkyl which is optionally substituted by chlorine and/or $C_1$-$C_3$-alkyl, or represents $C_5$- or $C_6$-cycloalkenyl, or represents benzyl which is optionally substituted by fluorine, chlorine and/or $C_1$-$C_4$-alkyl, or represents phenyl which is optionally substituted by fluorine, chlorine, bromine, iodine, cyano, nitro, $C_1$-$C_4$-alkyl, trifluoromethyl, $C_1$-$C_4$-alkoxy and/or $C_1$-$C_4$-alkylthio, or represents $C_1$-$C_8$-alkoxy which is optionally substituted by $C_1$-$C_4$-alkoxy, or
$R^1$ and $R^2$, together with the nitrogen atom to which they are bonded, form a saturated or unsaturated five- to seven-membered nitrogen heterocyclic radical which is optionally substituted by one to three $C_1$-$C_3$-alkyl radicals and is optionally benzo-fused.

2. Halogenated thiadiazolyl-oxyacetamides of the general formula (I) according to Claim 1, characterised in that, in this formula,
$R^1$ represents $C_1$-$C_4$-alkyl, allyl or propargyl,
$R^2$ represents $C_1$-$C_6$-alkyl, $C_1$-$C_2$-alkoxy-$C_1$-$C_2$-alkyl, allyl, propargyl, cyclopentyl, cyclohexyl, cyclohexenyl, benzyl, phenyl (which is optionally substituted by fluorine, chlorine, bromine, cyano, nitro, methyl, ethyl, trifluoromethyl, methoxy or ethoxy), $C_1$-$C_6$-alkoxy or $C_1$-$C_2$-alkoxy-$C_1$-$C_2$alkoxy, or
$R^1$ and $R^2$, together with the nitrogen atom to which they are bonded, represent piperidinyl which is optionally substituted by one to three methyl and/or ethyl radicals, or represent pyrrolidinyl which is optionally substituted by one or two methyl and/or ethyl radicals, or represent perhydroazepinyl, or represent 1,2,3,4-tetrahydro-quinolinyl.

3. Process for the preparation of halogenated thiadiazolyl-oxyacetamides of the general formula (I) according to Claim 1, characterised in that thiadiazole derivatives of the general formula (II)

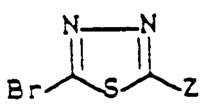

(II)

in which
Z represents a nucleofugic leaving group,
are reacted with hydroxyacetamides of the general formula (III)

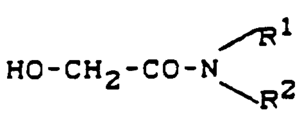

(III)

in which
$R^1$ and $R^2$ have the meanings given in Claim 1, if appropriate in the presence of a diluent, if appropriate

in the presence of an acid-binding agent and if appropriate in the presence of a catalyst.

4. Herbicidal agents, characterised in that they contain halogenated thiadiazolyl-oxyacetamides of the formula (I) according to Claim 1.

5. Method of combating undesirable plant growth, characterised in that halogenated thiadiazolyl-oxyacetamides of the formula (I) according to Claim 1 are allowed to act on the undesirable plants or their environment.

6. Use of halogenated thiadiazolyl-oxyacetamides of the formula (I) according to Claim 1 for combating plant growth.

7. Process for the preparation of herbicidal agents, characterised in that halogenated thiadiazolyl-oxyacetamides of the formula (I) according to Claim 1 are mixed with extenders and/or surface-active agents.

**Revendications**

1. Thiadiazolyloxyacétamides halogénés de formule générale I

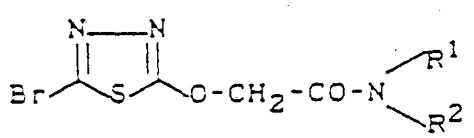

(I)

dans laquelle
$R^1$ représente l'hydrogène, un groupe alkyle en $C_1$-$C_8$ éventuellement substitué par le fluor, le chlore, des groupes cyano ou alcoxy en $C_1$-$C_4$, un groupe alcényle en $C_2$-$C_8$ éventuellement substitué par le fluor et/ou le chlore, un groupe alcynyle en $C_2$-$C_8$ ou benzyle,
$R^2$ représente un groupe alkyle en $C_1$-$C_8$ éventuellement substitué par le fluor, le chlore, des groupes cyano ou alcoxy en $C_1$-$C_4$, un groupe alcényle en $C_2$-$C_8$ éventuellement substitué par le fluor et/ou le chlore, un groupe alcynyle en $C_2$-$C_8$, un groupe cycloalkyle en $C_3$-$C_6$ éventuellement substitué par le chlore et/ou des groupes alkyle en $C_1$-$C_3$, un groupe cycloalcényle en $C_5$ ou $C_6$, un groupe benzyle éventuellement substitué par le fluor, le chlore et/ou des groupes alkyle en $C_1$-$C_4$, un groupe phényle éventuellement substitué par le fluor, le chlore, le brome, l'iode, des groupes cyano, nitro, alkyle en $C_1$-$C_4$, trifluorométhyle, alcoxy en $C_1$-$C_4$ et/ou alkylthio en $C_1$-$C_4$, un groupe alcoxy en $C_1$-$C_8$ éventuellement substitué par un groupe alcoxy en $C_1$-$C_4$, ou bien
$R^1$ et $R^2$ forment ensemble et avec l'atome d'azote auquel ils sont reliés un hétérocycle azoté, saturé ou insaturé, de 5 à 7 chaînons, portant éventuellement un à trois substituants alkyle en $C_1$-$C_3$ et éventuellement condensé avec un noyau benzénique.

2. Thiadiazolyloxyacétamides halogénés de formule générale I de la revendication 1, caractérisés en ce que :
$R^1$ représente un groupe alkyle en $C_1$-$C_4$, allyle ou propargyle,
$R^2$ représente un groupe alkyle en $C_1$-$C_6$, (alcoxy en $C_1$-$C_2$)-alkyle en $C_1$-$C_2$, allyle, propargyle, cyclopentyle, cyclohexyle, cyclohexényle, benzyle, phényle, (éventuellement substitué par le fluor, le chlore, le brome, des groupes cyano, nitro, méthyle, éthyle, trifluorométhyle, méthoxy, éthoxy), un groupe alcoxy en $C_1$-$C_6$ ou (alcoxy en $C_1$-$C_2$)-alcoxy en $C_1$-$C_2$, ou bien
$R^1$ et $R^2$ forment ensemble et avec l'atome d'azote auquel ils sont reliés un groupe pipéridinyle portant éventuellement un à trois substituants méthyle et/ou éthyle, un groupe pyrrolidinyle portant éventuellement un ou deux substituants méthyle et/ou éthyle, un groupe perhydroazépinyle ou un groupe 1,2,3,4-tétrahydroquinoléinyle.

3. Procédé de préparation des thiadiazolyloxyacétamides halogénés de formule I de la revendication 1, caractérisé en ce que l'on fait réagir des dérivés du thiadiazole de formule générale II

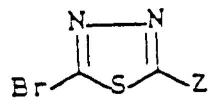

(II)

dans laquelle Z représente un groupe éliminable nucléofuge, avec des hydroxyacétamides de formule générale III

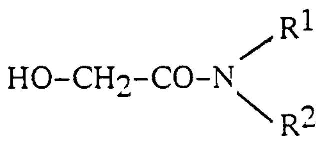

(III)

dans laquelle $R^1$ et $R^2$ ont les significations indiquées dans la revendication 1, éventuellement en présence d'un diluant, éventuellement en présence d'un accepteur d'acide et éventuellement en présence d'un catalyseur.

**4.** Produits herbicides, caractérisés en ce qu'ils contiennent des thiadiazolyloxyacétamides halogénés de formule générale I de la revendication 1.

**5.** Procédé pour combattre les croissances de végétaux indésirables, caractérisé en ce que l'on fait agir sur les végétaux indésirables ou leur habitat des thiadiazolyloxyacétamides halogénés de formule générale I de la revendication 1.

**6.** Utilisation des thiadiazolyloxyacétamides halogénés de formule générale I de la revendication 1 pour la lutte contre les croissances de végétaux.

**7.** Procédé de préparation de produits herbicides, caractérisé en ce que l'on mélange des thiadiazolyloxyacétamides halogénés de formule générale I de la revendication 1 avec des diluants et/ou des agents tensioactifs.